# EUROPÄISCHE PATENTSCHRIFT

(11) **EP 1 558 221 B1**
(45) Veröffentlichungstag und Bekanntmachung des Hinweises auf die Patenterteilung: **06.06.2007**
(21) Anmeldenummer: 03758053.7
(22) Anmeldetag: 24.10.2003
(51) Int. Cl.: A61K 9/28, A61K 9/20, A61K 9/14, A61K 31/485, A61K 31/4725

(54) **GEGEN MISSBRAUCH GESICHERTE DARREICHUNGSFORM**
DOSAGE FORM THAT IS SAFEGUARDED FROM ABUSE
FORME GALENIQUE PROTEGEE CONTRE UN USAGE DETOURNE

(30) Priorität: 25.10.2002 DE 10250087
(43) Veröffentlichungstag der Anmeldung: 03.08.2005
(73) Patentinhaber: Grünenthal GmbH, 52078 Aachen (DE)
(72) Erfinder: BARTHOLOMÄUS, Johannes, 52080 Aachen (DE); LANGNER, Klaus-Dieter, 52078 Aachen (DE)
(74) Vertreter: Kutzenberger, Helga
(86) Internationale Anmeldenummer: PCT/EP2003/011789
(87) Internationale Veröffentlichungsnummer: WO 2004/037230

(56) Entgegenhaltungen:
- WO-A-97/33566
- WO-A-03/013538
- US-A- 4 175 119
- US-A- 4 529 583

## Beschreibung

Die vorliegende Erfindung betrifft eine gegen Mißbrauch gesicherte, feste Darreichungsform umfassend wenigstens einen Wirkstoff mit Mißbrauchspotential und wenigstens ein davon räumlich getrenntes Emetikum, wobei der Wirkstoff bzw. die Wirkstoffe in wenigstens einer Untereinheit (a) und das Emetikum in wenigstens einer Untereinheit (b) vorliegen und das Emetikum aus der Untereinheit (b) bei bestimmungsgemäßer Applikation der Darreichungsform im Körper praktisch nicht freigesetzt wird.

Eine Vielzahl von pharmazeutischen Wirkstoffen weist neben einer ausgezeichneten Wirksamkeit auf ihrem betreffenden Anwendungsgebiet auch ein Mißbrauchspotential auf, d.h. sie können von einem Mißbraucher eingesetzt werden, um Wirkungen herbeizuführen, die nicht ihrem medizinischen Bestimmungszweck entsprechen.
So werden beispielsweise Opiate, die eine exzellente Wirksamkeit bei der Bekämpfung von starken bis sehr starken Schmerzen zeigen, von Mißbrauchern häufig zum Erzielen rauschartiger, euphorisierender Zustände verwendet.

Orale Darreichungsformen, die solche Wirkstoffe mit Mißbrauchspotential enthalten, führen üblicherweise selbst bei der Einnahme mißbräuchlich hoher Mengen nicht zu dem vom Mißbraucher gewünschten Ergebnis, da die Wirkstoffe im Blut nur langsam anfluten. Um dennoch einen Mißbrauch zu ermöglichen, werden die entsprechenden Darreichungsformen vom Mißbraucher zerkleinert, z.B. gemörsert, und z.B. durch Schnupfen über die Nase appliziert. Bei einer weiteren Form des Mißbrauchs wird der Wirkstoff aus dem durch Zerkleinerung der Darreichungsform erhaltenen Pulver mit Hilfe einer vorzugsweise wäßrigen Flüssigkeit extrahiert und die resultierende Lösung, ggf. nach Filtration durch Watte oder Zellstoff, parenteral, insbesondere intravenös, appliziert. Bei diesen Formen der Applikation kommt zu einem gegenüber - der oralen Applikation beschleunigten Anfluten des Wirkstoffes mit dem vom Mißbraucher gewünschten Ergebnis.

Die Aufgabe der vorliegenden Erfindung bestand daher darin, eine Darreichungsform für Wirkstoffe mit Mißbrauchspotential zur Verfügung zu stellen, die bei bestimmungsgemäßer Applikation dessen therapeutische Wirkung gewährleistet, bei einer mißbräuchlichen Einnahme jedoch nicht die vom Mißbraucher gewünschte Wirkung entfaltet.

Diese Aufgabe wurde durch die erfindungsgemäße, gegen Mißbrauch gesicherte, feste Darreichungsform gelöst, die wenigstens einen Wirkstoff mit Mißbrauchspotential und wenigstens ein davon räumlich getrenntes Emetikum umfaßt, wobei der Wirkstoff bzw. die Wirkstoffe in wenigstens einer Untereinheit (a) und das Emetikum in wenigstens einer Untereinheit (b) vorliegen und das Emetikum aus der Untereinheit (b) bei bestimmungsgemäßer Applikation der Darreichungsform im Körper praktisch nicht freigesetzt wird.

Untereinheiten im Sinne der vorliegenden Erfindung sind feste Formulierungen, die jeweils neben üblichen, dem Fachmann bekannten Hilfsstoffen nur den (die) Wirkstoff(e) oder nur das (die) Emetikum (Emetika) aufweisen. Methoden zur Herstellung entsprechender Untereinheiten sind dem Fachmann bekannt, beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers.

Die erfindungsgemäße Darreichungsform kann in ihren jeweiligen Untereinheiten (a) bzw. (b) jeweils einen oder mehrere Wirkstoffe mit Mißbrauchspotential sowie eines oder mehrere Emetika aufweisen. Vorzugsweise weist die erfindungsgemäße Darreichungsform in den entsprechenden Untereinheiten jeweils nur einen Wirkstoff und nur ein Emetikum auf.

Pharmazeutische Wirkstoffe mit Mißbrauchspotential sind, ebenso wie deren einzusetzende Mengen und Verfahren zu ihrer Herstellung, dem Fachmann an sich bekannt und können als solche in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Die erfindungsgemäße Darreichungsform eignet sich besonders zur Verhinderung des Mißbrauchs eines pharmazeutischen Wirkstoffs, der ausgewählt ist aus der Gruppe bestehend aus Opiaten, Opioiden, Tranquillantien, vorzugsweise Benzodiazepinen, Stimulantien und weiteren Betäubungsmitteln. Besonders geeignet ist die erfindungsgemäße Darreichungsform zur Verhinderung des Mißbrauchs von Opiaten, Opioiden, Tranquillantien sowie weitere Betäubungsmitteln, die ausgewählt sind aus der Gruppe bestehend aus N-{ 1-[2-(4-Ethyl-5-oxo-2-tetrazolin-1-yl)ethyl]-4-methoxymethyl-4-piperidyl} propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Bart ), Benzylmorphin, Bezitramid, 7-Brom-5-(2-pyridyl)-1*H*-1,4-benzodiazepin-2(3*H*)-on (Bromazepam), 2-Brom-4-(2-chlorphenyl)-9-methyl-6*H*-thieno[3,2-*f*][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-*endo*-ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1*S*,2*S*)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H-*1,4-benzodiazepin-2(3*H*)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H-*thieno[2,3-e][1,4]diazepin-2(3*H*)-on (Clotiazepam), 10-Chlor-11b-(2chlorphenyl)-2,3,7,11b-tetrahydrooxazolo[3,2-*d*][1,4]benzodiazepin-6(5*H*-on (Cloxazolam), (-)-Methyl-[3β-benzoyloxy-2β(1αH,5α*H*)-tropancarboxylat] (Cocain), 4,5α-Epoxy-3-methoxy-17-methyl-7-morphinen-6α-ol (Codein), 5-(1-Cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthlambuten, Dioxaphetylbutyrat. Dipipanon, (6a*R*,10e*R*)-6,6,9-Trimethyl-3-pentyl-6a,7,8,10a-tetrahydro-6*H*-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethyllofiazepat), 4,5α-Epoxy-3-ethoxy-17-methyl-7-morphtnen-6α-ol (Ethylmorphin), Etonltazen, 4,5α-Epoxy-7α-(1-hydroxy-1-methylbutyl)-6-methoxy-17-methyl-6,14-endo-ethano-morphinan-3-ol (Etorphin), *N-*Ethyl-3-phenyl-8,9,10-trinorborman-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7 -Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2d][1,4]benzodiazepin-4,7(6*H*)-dion (Ketazolam), 1-[4-(3-Hydroxyphenyl)-1-methyl-4-piperidyl]-1-propanon (Ketobemidon), (3S,6S)-6-Dimethylamino-4,4-dilphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol). Levophenacylmorphan, Lofentanil, 6-(2-Chlorpheny)-2-(4-methyl-1-piperazinylmethylen)-8-nitro-2*H-*imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-a]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-*o*-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-a][1,4]benzodiazepin (Midazolam), 2-(Benzhydrylsulfinyl)acetamid (Modafinil), 4,5a-Epoxy-17-methyl-7-morphinen-3,6α-diol (Morphin), Myrophin, (±)*-trans-*3-(1,1-Dimethylheptyl)-7,8,10,10α-tetrahydro-1-hydroxy-6,6-dimethyl-6*H*-dibenzo [*b*, *d*]pyran-9(6α*H*)-on (Nabilon), Nalbuphen, Nalorphin, Narcein, Nicomorphin, 1-Methyl-7-nitro-5-phenyt-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis-trans*)*-*10-Chlor-2,3,7,11b-tetrahydro-2-methyl-11b-phenyloxazolo[3,2-*d*][1,4]benzodiazepin-6-(5*H*)-on (Oxazolam), 4,5α-Epoxy-14-hydroxy-3-methoxy-17-methyt-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pemolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat}(Remifentanil), 5-sec-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1 R*,2R*)-3-(3-Dimethylamino-1-ethyl-2-methyl-propyl)-phenol, (1 R, 2R, 4S)-2-[Dimethylamino)methyl-4-(p-fluorbenzyloxy)-1-(m-methoxyphenyl)cyclohexanol, jeweils ggf. in Form entsprechender stereoisomerer Verbindungen sowie entsprechender Derivate, insbesondere Ester oder Ether, und jeweils physiologisch verträglicher Verbindungen, insbesondere Salze und Solvate.

Die Verbindungen (1 R*,2R*)-3-(3-Dimethylamino-1-ethyl-2-methyl-propyl)-phenol und (1R, 2R, 4S)-2-[Dimethylamino)methyl-4-(p-fluorbenzyloxy)-1-(m-methoxyphenyl)cyclohexanol, deren physiologisch verträglichen Verbindungen, insbesondere deren Hydrochloriden sowie Verfahren zu ihrer Herstellung sind beispielsweise aus EP-A-693475 bzw. EP-A-780369 bekannt.

Die erfindungsgemäße Darreichungsform eignet sich auch zur Verhinderung des Mißbrauchs von Stimulanzien, bevorzugt solcher, die ausgewählt sind aus der Gruppe bestehend aus Amphetamin, Norpseudoephedrin, Methylphenidat und jeweils ggf. deren entsprechenden physiologisch verträglichen Verbindungen, insbesondere deren Basen, Salzen und Solvaten.

Geeignete Emetika zur Verhinderung des Mißbrauchs der Wirkstoffe sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Vorzugsweise kommt in der erfindungsgemäßen Darreichungsform ein Emetikum auf Basis eines oder mehrerer Inhaltsstoffe von Radix Ipecacuanhae (Brechwurzel), vorzugsweise auf Basis des Inhaltsstoffes Emetin, zum Einsatz, wie sie z.B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden.

Vorzugsweise weist die erfindungsgemäße Darreichungsform als Emetikum Emetin in einer Menge von≥10 mg, besonders bevorzugt ≥20 mg und ganz besonders bevorzugt ≥ 40 mg pro Darreichungsform, d.h. Dosiereinheit auf.

Ebenfalls bevorzugt kommt als Emetikum Apomorphin in den erfindungsgemäßen Darreichungsformen zum Einsatz, insbesondere in solchen, die zur Verhinderung des parenteralen oder nasalen Mißbrauchs besonders geeignet sind.

Sofern als Emetikum Apomorphin in der erfindungsgemäßen Darreichungsform vorliegt, beträgt die jeweilige Mmenge pro Dosiereinheit vorzugsweise ≥ 3 mg, besonders bevorzugt ≥ 5 mg und ganz besonders bevorzugt ≥ 7 mg.

Ein wesentlicher Aspekt der vorliegenden Erfindung besteht darin, daß das Emetikum aus der Untereinheit bzw. den Untereinheiten (b) der erfindungsgemäßen Darreichungsform bei bestimmungsgemäßer Applikation im Körper praktisch nicht freigesetzt wird, nur in so geringen Mengen freigesetzt wird, daß es keine den Patienten beeinträchtigende Wirkung entfaltet oder bei der Passage durch den Körper des Patienten nur an solchen Freisetzungsorten abgegeben wird, an denen eine für eine Wirksamkeit ausreichende Resorption des Emetikums nicht gegeben ist. Vorzugsweise wird das Emetikum im Körper praktisch nicht freigesetzt.

Der Fachmann versteht, daß diese vorstehend genannten Bedingungen in Abhängigkeit von dem jeweils eingesetzten Emetikum und der Formulierung der Untereinheit (b) bzw. der Darreichungsform variieren können. Die für das jeweilige Emetikum optimale Formulierung kann durch einfache Vorversuche ermittelt werden.

Wird die erfindungsgemäße Darreichungsform zum Zwecke der mißbräuchlichen Einnahme des Wirkstoffes manipuliert, z.B. durch zermörsern und ggf. extrahieren des so erhaltenen Pulvers mit einem geeigneten Extraktionsmittel, wird neben dem Wirkstoff auch das Emetikum in einer Form erhalten, in der es von dem Wirkstoff nicht auf einfache Weise zu separieren ist, so daß es bei der Applikation der manipulierten Darreichungsform, insbesondere bei oraler und/oder parenteraler Verabreichung, seine Wirkung im Körper entfaltet und eine Abwehrreaktion des Körpers, nämlich starke Übelkeit bis hin zum Erbrechen, hervorruft und so den Mißbrauch der Darreichungsform verhindert.

Die Formulierung der erfindungsgemäßen Darreichungsform kann in vielfältiger Art und Weise nach üblichen, dem Fachmann bekannten Methoden erfolgen, wobei die Untereinheiten (a) und (b) in der erfindungsgemäßen Darreichungsform jeweils in beliebiger räumlicher Anordnung zueinander vorliegen können, sofern die vorstehend genannten Bedingungen für die Freisetzung des Emetikums erfüllt sind. Methoden zur Herstellung der Darreichungsformen sind dem Fachmann bekannt, beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers.

In einer bevorzugten Ausführungsform der erfindungsgemäßen Darreichungsform liegen beide Untereinheiten (a) und (b) in multipartikulärer Form vor, wobei Mikrotabletten, Mikrokapseln, Mikropellets, Granulaten, Sphäroiden, Perlen oder Pellets besonders bevorzugt sind und sowohl für die Untereinheit (a) als auch (b) dieselbe Form, d.h. Gestaltung gewählt wird, damit keine Separierung der Untereinheiten (a) von (b) durch mechanische Auslese möglich ist. Die multipartikulären Formen weisen bevorzugt eine Größe im Bereich von 0,1 bis 3 mm, besonders bevorzugt von 0,5 bis 2 mm auf.

Die Untereinheiten (a) und (b) in multpartikulärer Form können auch bevorzugt in eine Kapsel abgefüllt, in einer Flüssigkeit oder einem Gel suspendiert oder zu einer Tablette verpreßt werden, wobei die jeweiligen Endformulierungen dergestalt erfolgen, daß die Untereinheiten (a) und (b) auch in der resultierenden Darreichungsform erhalten bleiben.

Die jeweiligen multipartikulären Untereinheiten (a) bzw (b) mit identischer Formgebung dürfen auch nicht visuell voneinander unterscheidbar sein, damit sie vom Mißbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z.B. die Retardierung eines oder mehrerer Wirkstoffe oder eine magensaftresistente Ausrüstung der jeweiligen Untereinheiten.

In einer weiteren bevorzugten Ausführungsform der vorliegenden Erfindung sind die Untereinheiten (a) und (b) jeweils schichtförmig zueinander angeordnet.
Bevorzugt sind hierfür die schichtförmigen Untereinheiten (a) und (b) in der erfindungsgemäßen Darreichungsform vertikal oder horizontal zueinander angeordnet, wobei jeweils auch eine oder mehrere schichtförmige Untereinheiten (a) und eine oder mehrere schichtförmige Untereinheiten (b) in der Darreichungsform vorliegen können, so daß neben den bevorzugten Schichtenfolgen (a)-(b) bzw. (a)-(b)-(a) beliebige andere Schichtenfolgen in Betracht kommen.

Ebenfalls bevorzugt ist eine erfindungsgemäße Darreichungsform, in der die Untereinheit (b) einen Kern bildet, der von der Untereinheit (a) vollständig umhüllt wird, wobei zwischen diesen Schichten eine ggf. quellbare Trennschicht (c) vorhanden sein kann. Ein entsprechender Aufbau eignet sich bevorzugt auch für die obenstehend genannten multipartikulären Formen, wobei dann beide Untereinheiten (a) und (b) sowie eine ggf. vorhandene Trennschicht (c) in ein- und derselben multipartikulären Form formuliert sind.

In einer weiteren bevorzugten Ausführungsform der erfindungsgemäßen Darreichungsform bildet die Untereinheit (a) einen Kern, der von der Untereinheit (b) umhüllt wird, wobei letztere wenigstens einen Kanal aufweist, der von dem Kern an die Oberfläche der Darreichungsform führt.

Zwischen einer Schicht der Untereinheit (a) und einer Schicht der Untereinheit (b) kann die erfindungsgemäße Darreichungsform jeweils eine oder mehrere, vorzugsweise eine, ggf. quellbare Trennschicht (c) zur räumlichen Trennung der Untereinheit (a) von (b) aufweisen.

Sofern die erfindungsgemäße Darreichungsform die schichtförmigen Untereinheiten (a) und (b) sowie eine ggf. vorhandene Trennschicht (c) in einer zumindest teilweise vertikalen oder horizontalen Anordnung aufweist, liegt sie bevorzugt in Form einer Tablette, eines Coextrudats oder Laminats vor.

Hierbei kann in einer besonders bevorzugten Ausführungsform die freie Oberfläche der Untereinheit (b) vollständig und ggf. zumindest ein Teil der freien Oberfläche der Untereinheit(en) (a) und ggf. zumindest ein Teil der freien Oberfläche der ggf. vorhandenen Trennschicht(en) (c) mit wenigstens einer die Freisetzung des Emetikums verhindernden Barriereschicht (d) überzogen sein.

Eber is besonders bevorzugt ist eine Ausführungsform der erfindungsgemäßen Darreichungsform, die eine vertikale oder horizontale Anordnung der Schichten der Untereinheiten (a) und (b) und wenigstens eine dazwischen angeordnete Push-Schicht (p) sowie ggf. eine Trennschicht (c) aufweist, in der sämtliche freie Oberflächen des aus den Untereinheiten (a) und (b), der Push-Schicht (p) und ggf. vorhandener Trennschicht (c) bestehenden Schichtaufbaus mit einem semipermeablen Überzug (e) ausgerüstet sind, der für ein Freisetzungsmedium, d.h. üblicherweise eine physiologische Flüssigkeit, durchlässig, für den Wirkstoff und für das Emetikum im wesentlichen undurchlässig ist, und wobei dieser Überzug (e) im Bereich der Untereinheit (a) wenigstens eine Öffnung zur Freisetzung des Wirkstoffes aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt.

In einer weiteren bevorzugten Ausführungsform hat die Untereinheit (a) der erfindungsgemäßen Darreichungsform die Form einer Tablette, deren Steg und ggf. eine der beiden Grundflächen mit einer das Emetikum enthaltenden Barriereschicht (d) bedeckt ist.

Der Fachmann versteht, daß die bei der Formulierung der erfindungsgemäßen Darreichungsform jeweils zum Einsatz kommenden Hilfsstoffe der Untereinheit(en) (a) bzw. (b) sowie ggf. der vorhandenen Trennschicht(en) (c) und/oder der Barriereschicht(en) (d) in Abhängigkeit von deren Anordnung in der erfindungsgemäßen Darreichungsform, der Applikationsart sowie in Abhängigkeit von dem jeweiligen Wirkstoff und dem Emetikum variieren. Die Materialien, die über die jeweils erforderlichen Eigenschaften verfügen sind, dem Fachmann an sich bekannt.

Sofern die Freisetzung des Emetikums aus der Untereinheit (b) der erfindungsgemäßen Darreichungsform mit Hilfe einer Umhüllung, vorzugsweise einer Barriereschicht, verhindert wird, kann die Untereinheit aus üblichen, dem Fachmann bekannten Materialien bestehen.

Ist eine entsprechende Barriereschicht (d) zur Verhinderung der Freisetzung des Emetikums nicht vorgesehen, sind die Materialien der Untereinheiten so zu wählen, daß eine Freisetzung des Emetikums aus der Untereinheit (b) praktisch ausgeschlossen ist.
Bevorzugt können hierzu die nachstehend aufgeführten Materialien zum Einsatz kommen, die auch für den Aufbau der Barriereschicht geeignet sind:

Bevorzugte Materialien können ausgewählt werden aus der Gruppe bestehend aus Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geignete Materialien können ausgewählt werden aus der Gruppe bestehend aus Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe bestehend aus Copolymeren aus Butylmethacrylat und Isobutylmethacrylat, Copolymeren aus Methylvinylether und Maleinsäure mit erhöhtem Molekulargewicht, Copolymeren aus Methylvinylether und Maleinsäuremonoethylester, Copolymeren aus Methylvinylether und Maleinsäureanhydrid sowie Copolymeren aus Vinylalkohol und Vinylacetat.

Weitere, zur Formulierung der Barriereschicht besonders geeignete, biologisch abbaubare Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1). Ggf. können die vorstehend genannten Materialien mit weiteren üblichen, dem Fachmann bekannten Hilfsstoffen, vorzugsweise ausgewählt aus der Gruppe bestehend aus Glycerylmonostearat, halbsynthetischen Triglyceridderivaten, halbsynthetischen Glyceriden, hydriertem Rizinusöl, Glycerylpalmitostearat, Glycerylbehenat, Polyvinylpyrrolidon, Gelatine, Magnesiumstearat, Stearinsäure. Natriumstearat, Talkum, Natriumbenzoat, Borsäure und kolloidalem Silica, Fettsäuren, substituierten Triglyceriden, Glyceriden, Polyoxyalkylenglykolen und deren Derivate abgemischt werden.

Sofern die erfindungsgemäße Darreichungsform eine Trennschicht (c) aufweist, kann diese, ebenso wie die nicht von einer Barriereschicht umhüllte Untereinheit (b) vorzugsweise aus den vorstehend, für die Barriereschicht beschriebenen Materialien bestehen. Der Fachmann versteht, daß auch über die Dicke der Trennschicht jegliche Freisetzung des Emetikums aus der jeweiligen Untereinheit gesteuert werden kann.

Die erfindungsgemäße Darreichungsform zur oralen Verabreichung eines oder mehrerer Wirkstoffe eignet sich besonders zur Verhinderung eines oralen, nasalen und/oder parenteralen Mißbrauchs solcher Wirkstoffe.

Dabei können auch ein oder mehrere Wirkstoffe zumindest teilweise in retardierter Form vorliegen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muß aber so gesteuert sein, daß bei bestimmungsgemäßer Applikation der Darreichungsform der Wirkstoff bzw. die Wirkstoffe praktisch komplett freigesetzt wird, bevor das Emetikum eine beeinträchtigende Wirkung entfalten kann.

Sofern die erfindungsgemäße Darreichungsform zur oralen Applikation vorgesehen ist, kann sie bevorzugt auch einen magensaftresistenten Überzug aufweisen, der sich in Abhängigkeit vom pH-Wert der Freisetzungsumgebung auflöst.
Durch diesen Überzug kann erreicht werden, daß die erfindungsgemäße Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf. Die Untereinheit (b) ist dann vorzugsweise so zu formulieren, daß das Emetikum im Körper praktisch nicht freigesetzt wird.

Entsprechende Materialien und Verfahren zur Retardierung von Wirkstoffen sowie zum Aufbringen magensaftresistenter Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt. Die entsprechende Literaturbeschreibung wird hiermit als Referenz eingeführt und gilt als Teil der Offenbarung.
In einer weiteren bevorzugten Ausführungsform ist die Menge des Emetikums in der erfindungsgemäßen Darreichungsform so gewählt, daß bei bestimmungsgemäßer oraler Applikation keine negative Wirkung hervorgerufen wird. Wird jedoch die vorgesehene Dosierung der Darreichungsform versehentlich, insbesondere durch Kinder, oder beim Mißbrauch überschritten, wird Übelkeit bzw. Brechreiz hervorgerufen. Die jeweilige Menge des Emetikums, die vom Patienten bei bestimmungsgemäßer oraler Applikation noch toleriert wird, kann vom Fachmann durch einfache Vorversuche ermittelt werden.

Die erfindungsgemäßen Darreichungsformen haben den Vorteil, daß sie gegen nasalen und/oder parenteralen sowie ggf. auch gegen oralen Mißbrauch geschützt sind, ohne daß bei bestimmungsgemäßer Applikation eine Beeinträchtigung des zu therapierenden Patienten oder eine Verminderung der Wirksamkeit des jeweiligen Wirkstoffes zu befürchten ist. Sie lassen sich einfach und vergleichsweise kostengünstig produzieren.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die nachstehenden Mengenangaben beziehen sich jeweils auf eine Darreichungsform. Eine Charge eines Herstellgangs bestand aus 1000 Darreichungsformen.

### Beispiel 1

### Manteltabletten

### Kern

| | |
|---|---|
| Emetin | 50 mg |
| Hydriertes Rizinusöl (Cutina HR) | 50 mg |

Emetin und fein gepulvertes Hydriertes Rizinusöl wurden gemischt und auf einer Tablettenpresse zu runden, bikonvexen Tabletten mit 6,5 mm Durchmesser verpreßt.

### Mantel

| | |
|---|---|
| Morphinsulfat Pentahydrat | 60 mg |
| Methylhydroxypropylcellulose 100 000 mPas (Metolose 90 SH 100 000, ShinEtsu) | 100 mg |
| Mikrokristalline Cellulose (Avicel PH 102) | 165 mg |
| Lactose Monohydrat | 165 mg |
| Magnesiumstearat | 5 mg |
| Kolloidales Siliciumdioxid | 5 mg |

Alle Mantelbestandteile wurden gemischt, in einer Tablettenpresse mit Werkzeug für 13 mm bikonvexe Tabletten werden ca. 250 mg der Mischung in die Tablettenmatrize gefüllt, der 6,5 mm Kern zentriert eingelegt, die restlichen 250 mg der Mantelmischung zugefüllt und der Mantel um den Kern gepreßt.

### Beispiel 2

### Manteltabletten

### Kern

| | |
|---|---|
| Emetin | 50 mg |
| Hydriertes Rizinusöl (Cutina HR) | 50 mg |

Emetin und fein gepulvertes Hydriertes Rizinusöl wurden gemischt und auf einer Tablettenpresse zu runden, bikonvexen Tabletten mit 6,5 mm Durchmesser verpreßt.

### Mantel

| | |
|---|---|
| Oxycodon-Hydrochlorid | 30 mg |
| Srühgetrocknete Lactose | 300 mg |
| Eudragit RSPM | 70 mg |
| Stearylakohol | 115 mg |
| Magnesiumstearat | 5 mg |
| Talkum | 10 mg |

Oxycodon-Hydrochlorid, sprühgetrocknete Lactose und Eudragit RSPM wurden in einem geeigneten Mischer ca. 5 min innig miteinander vermischt. Während des Mischens wurde die Mischung mit einer solchen Menge Gereinigtem Wasser granuliert, daß sich eine feuchte granulierte Masse bildet. Das so erhaltene Granulat wurde in der Wirbelschicht bei 60°C getrocknet und durch ein 2,5 mm Sieb gesiebt. Anschließend wurde das Granulat erneut wie vor beschrieben getrocknet und durch ein 1,5 mm Sieb gesiebt. Der Stearylakohol wurde bei 60-70°C geschmolzen und in einem Mischer zu dem Granulat gegeben. Nach dem Abkühlen wurde die Masse durch ein 1,5 mm Sieb gesiebt. Vom so erhaltenen Granulat wurde in einer Tablettenpresse mit Werkzeug für 13 mm bikonvexe Tabletten ca. 265 mg der Mischung in die Tablettenmatrize gefüllt, der 6,5 mm Kern zentriert eingelegt, die restlichen 265 mg der Mantelmischung zugefüllt und der Mantel um den Kern gepreßt.

### Beispiel 3

### Manteltabletten

### Kern

| | |
|---|---|
| Emetin | 50 mg |
| Sprühgetrocknete Lactose | 46 mg |
| Magnesiumstearat | 2 mg |
| Kolloidales Siliciumdioxid | 2 mg |

Alle Bestandteile wurden gemischt und auf einer Tablettenpresse zu runden, bikonvexen Tabletten mit 6,5 mm Durchmesser verpreßt.

### Überzug auf Kern

| | |
|---|---|
| Celluloseacetat mit 39,8 % Acetat | 9,5 mg |
| Macrogol 3350 | 0,5 mg |

Die Überzugsbestandteile wurden in einem Aceton-Wasser Gemisch (95:5 Gewichtsteile) gelöst und auf die Kerne gesprüht.

### Mantel

| | |
|---|---|
| Morphinsulfat Pentahydrat | 60 mg |
| Methylhydroxypropylcellulose 100 000 mPas (Metolose 90 SH 100 000, ShinEtsu) | 100 mg |
| Mikrokristalline Cellulose (Avicel PH 102) | 165 mg |
| Lactose Monohydrat | 165 mg |
| Magnesiumstearat | 5 mg |
| Kolloidales Siliciumdioxid | 5 mg |

Alle Mantelbestandteile wurden gemischt, in einer Tablettenpresse mit Werkzeug für 13 mm bikonvexe Tabletten wurden ca. 250 mg der Mischung in die Tablettenmatrize gefüllt, der mit Celluloseacetat überzogene Kern zentriert eingelegt, die restlichen 250 mg der Mantelmischung zugefüllt und der Mantel um den Kern gepreßt.

### Beispiel 4

### Multipartikuläre Form

### Emetika Pellets

| | |
|---|---|
| Emetin | 50 mg |
| Lactose | 15 mg |
| Mikrokristalline Cellulose PH101 | 30 mg |
| Niedrigsubstituierte Hydroxypropylcellulose (LH31, Shin-Etsu) | 5 mg |

Alle Bestandteile wurden in einem geeigneten Mischer ca. 5 min innig miteinander vermischt. Während des Mischens wurde die Mischung mit einer solchen Menge Gereinigtem Wasser granuliert, daß sich eine feuchte granulierte Masse bildete. Das so erhaltene Granulat wurde in einem Nica-Extruder durch eine Matrize mit Extrusionsöffnungen von 1 mm extrudiert, 5 min auf einem Spheronizer gerundet, in der Wirbelschicht bei 60°C getrocknet und mittels eines 1,5 mm und eines 0,5 mm Siebs klassiert.

### Überzug auf Emetika Pellets

| | |
|---|---|
| Celluloseacetat mit 39,8 % Acetat | 9,5 mg |
| Macrogol 3350 | 0,5 mg |
| Titandioxid | 0,5 mg |

Mengenangaben pro 100 mg Emetikapellets

Celluloseacetat und Macrogol wurden in einem Aceton-Wasser Gemisch (95:5 Gewichtsteile) gelöst, Titandioxid wird in der Mischung dispergiert und die Kerne in einer Wirbelschichtanlage mit der Suspension besprüht bis die Masse der überzogenen Pellets 110% des Gewichts der eingesetzten nicht überzogenen Pellets betrug.

### Analgetika Pellets

| | |
|---|---|
| Nonpareils 0,5 mm (Saccharose-Maisstärke Starter Pellets, Fa. Werner) | 50 mg |
| Morphinsulfat Pentahydrat | 60 mg |
| Povidon K 30 | 30 mg |
| Talkum | 10 mg |

Morphinsulfat und Povidon wurden in Gereinigtem Wasser gelöst und Talkum wird in der Lösung dispergiert. Die Suspension wurde bei 60°C auf die Nonpareils aufgesprüht und getrocknet. Die Pellets wurden mittels eines 1,5 mm und eines 0,5 mm Siebs klassiert.

### Überzug auf Analgetica Pellets

| | |
|---|---|
| Ethylcellulose Dispersion (Aquacoat ECD30, FMC Corporation) | 10,0 mg |
| Glycerolmonostearat | 2,0 mg |
| Talkum | 2,0 mg |
| Titandioxid | 1,0 mg |

Mengenangaben pro 150 mg Analgetica Pellets, Ethylcellulosegewichtsangabe als Trockenmasse aus der 30%igen Dispersion des Handelsprodukts.

Ethylcellulose Dispersion wurde 1:0,5 mit Gereinigtem Wasser gemischt und das Glycerolmonostearat unter mindestens zwei stündigem Rühren eingearbeitet. Talkum und Titandioxid wurden in 0,5 Teilen Wasser (Berechnungsbasis aus der 1:0,5 Mischung der Ethylcellulose Dispersion) dispergiert und mit der Ethylcellulosedispersion gemischt. Die Analgetica Pellets wurden in einer Wirbelschichtanlage mit der Dispersion besprüht bis die Masse der überzogenen Pellets 110% des Gewichts der eingesetzten nicht überzogenen Pellets betrug.

### Endformulierung in Kapseln

Pro Kapsel wurden jeweils 110 mg überzogene Emetika Pellets und 165 mg überzogene Analgetica Pellets gemischt und in Hartgelatinekapseln der Größe 1 abgefüllt.

### Beispiel 5

### Manteltabletten

### Kern

| | |
|---|---|
| Emetinhydrochlorid-Pentahydrat | 60 mg |
| Hydriertes Rizinusöl (Cutina HR) | 40 mg |

Emetinhydrochlorid-Pentahydrat und fein gepulvertes Hydriertes Rizinusöl wurden gemischt und auf einer Tablettenpresse zu runden, bikonvexen Tabletten mit 6,5 mm Durchmesser verpreßt.

### Überzug auf Kern

| | |
|---|---|
| Celluloseacetat mit 39,8 % Acetat | 9,5 mg |
| Macrogol 3350 | 0,5 mg |

Die Überzugsbestandteile wurden in einem Aceton-Wasser Gemisch (95:5 Gewichtsteile) als 3,8% ige Lösung gelöst und auf die Kerne gesprüht.

### Mantel

| | |
|---|---|
| Morphinsulfat Pentahydrat | 60 mg |
| Methylhydroxypropylcellulose 100 000 mPas (Metolose 90 SH 100 000, ShinEtsu) | 100 mg |
| Mikrokristalline Cellulose (Avicel PH 102) | 165 mg |
| Lactose Monohydrat | 165 mg |
| Magnesiumstearat | 5 mg |
| Kolloidales Siliciumdioxid | 5 mg |

Alle Mantelbestandteile wurden gemischt, in einer Tablettenpresse mit Werkzeug für 13 mm bikonvexe Tabletten wurden ca. 250 mg der Mischung in die Tablettenmatrize gefüllt, der mit Celluloseacetat überzogene 6,5 mm Kern zentriert eingelegt, die restlichen 250 mg der Mantelmischung zugefüllt und der Mantel um den Kern gepreßt.

### Beispiel 6

### Orales osmotisches therapeutisches System (OROS)

### Wirkstoffschicht

| | |
|---|---|
| Morphinsulfat Pentahydrat | 125 mg |
| Macrogol 200 000 | 280 mg |
| Povidon (MG_{N} 40 000) | 26 mg |
| Magnesiumstearat | 4 mg |

Morphinsulfat und Macrogol wurden in einem Planetenmischer trocken gemischt und anschließend unter langsamer Zugabe einer Lösung des Povidon in 115 mg Ethanol zu einer feuchten Masse angeteigt, die dann durch ein 0,8 mm Sieb getrieben wurden. Nach 24 Stunden Trocknen bei Raumtemperatur in einem Abzug wurden die Partikel zusammen mit dem Magnesiumstearat durch ein 1,0 mm Sieb getrieben und in einem Containermischer gemischt.

### Push-Schicht

| | |
|---|---|
| Methylhydroxypropylcellulose 6 mPas | 13 mg |
| Natriumchlorid | 80 mg |
| Macrogol 7 000 000 | 166 mg |
| Magnesiumstearat | 1 mg |

Natriumchlorid, Macrogol und die Hälfte der Methylhydroxypropylcellulose wurden in einem Wirbelschichtgranulator 3 Minuten trocken gemischt und anschließend durch Aufsprühen einer Lösung der zweiten Hälfte der Methylhydroxypropylcellulose in 75 mg unter Zufuhr von Warmluft granuliert und getrocknet. Das Granulat wurde anschließend zusammen mit dem Magnesiumstearat durch ein 2,5 mm Sieb in einer Comil getrieben.

### Emetikaschicht

| | |
|---|---|
| Emetin | 50 mg |
| Hydriertes Rizinusöl (Cutina HR) | 50 mg |

Emetin und Hydriertes Rizinusöl wurden in einer Tablettenpresse mit einem 10 mm Vorpreßstempel zu Preßlingen von ca. 250 mg vorgepreßt. Anschließend wurde die Vorpreßlinge mittels eines Brechers und eines Siebes von 1,0 mm zerkleinert.

### Herstellung der 3-Schichttabletten

Pro Tablette wurden 100 mg des Granulates der Emetikaschicht, 260 mg der Push-Schicht und 435 mg der Wirkstoffschicht nacheinander in die Matrize einer geeigneten Tablettenpresse gefüllt und zu einer 3-Schichttablette verpreßt.

### Überzug auf Kern

| | |
|---|---|
| Celluloseacetat mit 39,8 % Acetat | 38 mg |
| Macrogol 3350 | 2 mg |

Die Überzugsbestandteile wurden in einem Aceton-Wasser Gemisch (95:5 Gewichtsteile) als 3,8% ige Lösung gelöst und auf die Kerne gesprüht. Durch den Überzug wurden zwei Löcher von 0,75 mm gebohrt um die Wirkstoffschicht mit der äußeren Umgebung des Systems zu verbinden.

### Beispiel 7

### Orales osmotisches therapeutisches System

Die Herstellung erfolgte analog zu Beispiel 6 mit dem Unterschied, daß die Emetikaschicht die folgende Zusammensetzung aufwies:

| | |
|---|---|
| Emetinhydrochlorid-Pentahydrat | 60 mg |
| Hydriertes Rizinusöl (Cutina HR) | 40 mg |

Emetinhydrochlorid-Pentahydrat und Hydriertes Rizinusöl werden in einer Tablettenpresse mit einem 10 mm Vorpreßstempel zu Preßlingen von ca. 250 mg vorgepreßt. Anschließend werden die Vorpreßlinge mittels eines Brechers und eines Siebes von 1,0 mm zerkleinert. Alle übrigen Herstellschritte erfolgen wie in Beispiel 6 dargestellt.

## Patentansprüche

1. Gegen Mißbrauch gesicherte, feste Darreichungsform umfassend wenigstens einen Wirkstoff mit Mißbrauchspotential und wenigstens ein davon räumlich getrenntes Emetikum, wobei der Wirkstoff bzw. die Wirkstoffe in wenigstens einer Untereinheit (a) und das Emetikum In wenigstens einer Untereinheit (b) vorliegen und das Emetikum aus der Untereinheit (b) bei bestimmungsgemäßer Applikation der Darreichungsform im Körper praktisch nicht freigesetzt wird.

2. Darreichungsform gemäß Anspruch 1, **dadurch gekennzeichnet, daß** der Wirkstoff bzw. die Wirkstoffe pharmazeutische Wirkstoffe aus der Gruppe bestehend aus Opiaten, Opioiden, Tranquillantien, vorzugsweise Benzodiazepinen, Stimulantien und weiteren Betäubungsmitteln ausgewählt worden sind.

3. Darreichungsform gemäß Anspruch 2, **dadurch gekennzeichnet, daß** der Wirkstoffe bzw. die Wirkstoffe ausgewählt worden sind aus der Gruppe bestehend aus N-{ 1-[2-(4-Ethyl-5-oxo-2-tetrazolin-1-yl)ethyl]-4-methoxymethyl-4-piperidyl}propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyrdyl)-1*H*-1,4-benzodiazepin-2(3*H*)-on (Bromazepam), 2-Brom-4-(2-chlorphenyl)-9-methyl-6*H*-thieno[3,2-*f*][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-*endo-*ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1*S*,2*S*)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H-*1,4-benzodiazepin-2(3*H*)-on (Clonazepam), Clonitazen. 7-Chlor-2,3-dihydro-2-oxo-5-phenyl-1*H*-1,4-benzadiazepin-3-carbonsäure (Clorazepat), 5-(2-Chlorphenyl)-7-ethyl-1-methyl-1*H*-thieno[2,3-e][1,4]diazepin-2(3*H*)-on (Clotiazepam), 10-Chlor-11b-(2chlorphenyl)-2,3,7,11b-tetrahydrooxazolo[3,2-*d*][1,4]benzodiazepin-6(5*H*)-on (Cloxazolam), (-)-Methyl-[3β-benzoyloxy-2β(1αH,5α*H*)-tropancarboxylat] (Cocain),4,5α-Epoxy-3-methoxy-17-methyl-7-morphinen-8α-ol (Codein), 5-(1-Cyclohexenyl)-5-ethylbarbitursäure (Cyclobarbital), Cyclorphan, Cyprenorphin, 7-Chlor-5-(2-cholorphenyl)-1*H*-1,4-benzodiazepin-2(3*H*)-on (Delorazepam), Desomorphin, Dextromoramid, (+)-(1-Benzyl-3-dimathylamino-2-methyl-1-phenylpropyl)proponat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein). 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10a*R*)-6,6,9-Trimethyl-3-pentyl-8a,7,8,10a-tetrahydro-6*H*-benzo[c]chromen-1-ol (Dronabinol), Eptazocin, 8-Chlor-6-phenyl-4*H*-[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H-*1,4 benzodiazepin-3-carboxylat] (Ethylloflazepat), 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*endo*-etheno-morphinan-3-ol (Etorphin), *N*-Ethyl-3-phenyl-8,9,10-trinorboman-2-ylamin (Fencamfamin), 7-[2-(α-Methylphenothylamino)ethyl]-theophylin) (Fenetyllin), 3-(α-Methylphenethylamino)propionitril (Fenproporex), *N*-(1-Phenethyl-4-piperidyl)propionanllid (Fentanyl), 7-Chlor-5-(2-fluorphenyl)-1-methyl-1*H*-1,4-benzodiazepin-2(3*H*)-on (Fludiazepam). 5-(2-Fluorphenyl)-1-methyl-7-nitro-1*H-*1,4-benzodiazepin-2(3*H*)-on (Flunitrazepam). 7-Chlor-1-(2-diethylaminoethyl)-5-(2-fluorphenyl)-1*H*-1,4-benzodiazepin-2(3*H*)-on (Flurazepam). 7 -Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydrox-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3S,6S)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-1-piperazinylmethylen)-8-nitro-2*H*-imidazo[1,2-a][1,4] benzodiazepin-1(4*H*)-on (Loprazolam), 7-Chlor-5-(2-chlorphenyl)3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-a]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-*o*-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-a][1,4]benzodiazepin (Midazolam), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H*-dibenzo [*b*, *d*]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis-trans*)-10-Chlor-2,3,7,11b-tetrahydro-2-methyl-11 b-phenyloxazolo[3,2-*d*][1,4] benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pemolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N*-phenylpropanamido)piperidino]propanoat} (Remifentanil), 5-*sec*-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)cyclohexanol, jeweils ggf. in Form entsprechender stereoisomerer Verbindungen sowie entsprechender Derivate, insbesondere Ester oder Ether, und jeweils physiologisch verträglicher Verbindungen, insbesondere Salze und Solvate.

4. Darreichungsform gemäß Anspruch 2 oder 3, **dadurch gekennzeichnet, daß** das Stimulanz ausgewählt worden ist aus der Gruppe bestehend aus Amphetamin, Norpseudoephedrin, Methylphenidat und jeweils ggf. deren entsprechenden physiologisch verträglichen Verbindungen, insbesondere deren Basen, Salzen und Solvaten.

5. Darreichungsform gemäß einem der Ansprüche 1 bis 4, **dadurch gekennzeichnet, daß** das Emetikum auf einem oder mehreren Inhaltsstoffen von Radix Ipecacuanhae (Brechwurzel), vorzugsweise auf dem Inhaltsstoff Emetin basiert, und/oder Apomorphin ist.

6. Darreichungsform gemäß einem der Ansprüche 1 bis 5, **dadurch gekennzeichnet, daß** beide der Untereinheiten (a) und (b) in multipartikulärer Form, vorzugsweise in Form von Mikrotabletten, Mikrokapseln, Mikropellets, Granulaten, Sphäroiden, Perlen oder Pellets, ggf. zu Tabletten verpreßt, in Kapseln abgefüllt oder in einer Flüssigkeit oder einem Gel suspendiert, vorliegen, wobei sowohl für die Untereinheit (a) als auch (b) dieselbe Form, d.h. Gestaltung gewählt wird.

7. Darreichungsform gemäß Anspruch 6, **dadurch gekennzeichnet, daß** die jeweiligen weitgehend identisch gestalteten multipartikulären Formen der Untereinheit (a) bzw (b) auch visuell nicht unterscheidbar sind.

8. Darreichungsform gemäß einem der Ansprüche 1 bis 5, **dadurch gekennzeichnet, daß** die Untereinheiten (a) und (b) jeweils schichtförmig zueinander angeordnet sind.

9. Darreichungsform gemäß Anspruch 8, **dadurch gekennzeichnet, daß** die schichtförmigen Untereinheiten (a) und (b) vertikal oder horizontal zueinander angeordnet sind.

10. Darreichungsform gemäß Anspruch 8, **dadurch gekennzeichnet, daß** die Untereinheit (b) einen Kern bildet, der von der Untereinheit (a) vollständig umhüllt wird.

11. Darreichungsform gemäß Anspruch 8, **dadurch gekennzeichnet, daß** die Untereinheit (a) einen Kern bildet, der von der Untereinheit (b) umhüllt wird, wobei diese Umhüllung wenigstens einen Kanal aufweist, der von dem Kern an die Oberfläche der Darreichungsform führt.

12. Darreichungsform gemäß einem der Ansprüche 8 bis 11, **dadurch gekennzeichnet, daß** zwischen den Schichten der Untereinheiten (a) und (b) wenigstens eine, ggf. quellbare Trennschicht (c) angeordnet ist.

13. Darreichungsform gemäß Anspruch 8, 9 oder 12, **dadurch gekennzeichnet, daß** sie in Form einer Tablette vorliegt.

14. Darreichungsform gemäß einem der Ansprüche 8, 9, 12 oder 13, **dadurch gekennzeichnet**, die freie Oberfläche der Untereinheit (b) vollständig und ggf. zumindest ein Teil der freien Oberfläche der Untereinheit (a) und ggf. zumindest ein Teil der freien Oberfläche der ggf. vorhandenen Trennschicht (c) mit wenigstens einer die Freisetzung des Emetikums verhindernden Barriereschicht (d) überzogen ist.

15. Darreichungsform gemäß einem der Ansprüche 8, 9, 12 oder 13, **dadurch gekennzeichnet, daß** sie zwischen den Untereinheiten (a) und (b) eine Push-Schicht (p) aufweist und sämtliche freie Oberflächen des Schichtaufbaus aus den Untereinheiten (a) und (b), der Push-Schicht (p) und ggf. der Trennschicht (c) mit einem semipermeablen Überzug (e) ausgerüstet sind, der für das Freisetzungsmedium durchlässig, für den Wirkstoff und für das Emetikum im wesentlichen undurchlässig ist, und wobei dieser Überzug (e) im Bereich der Untereinheit (a) wenigstens eine Öffnung zur Freisetzung des Wirkstoffes aufweist.

16. Darreichungsform gemäß einem der Ansprüche 1 bis 5, **dadurch gekennzeichnet, daß** die Untereinheit (a) die Form einer Tablette hat, deren Steg und ggf. eine der beiden Grundflächen mit wenigstens einer das Emetikum enthaltenden Barrierschicht (d) bedeckt ist.

17. Darreichungsform gemäß einem der Ansprüche 1 bis 16, **dadurch gekennzeichnet, daß** sie wenigstens einen Wirkstoff zumindest teilweise in retardierter Form aufweist.

18. Darreichungsform gemäß einem der Ansprüche 1 bis 17 zur oralen Verabreichung.

19. Darreichungsform gemäß Anspruch 18, **dadurch gekennzeichnet, daß** sie wenigstens einen magensaftresistenten Überzug aufweist.

## Claims

1. An abuse-proofed solid dosage form comprising at least one active ingredient with potential for abuse and at least one emetic spatially separate therefrom, wherein the active ingredient or active ingredients is/are present in at least one subunit (a) and the emetic is present in at least one subunit (b) and the emetic from subunit (b) is practically not released in the body when the dosage form is correctly administered.

2. A dosage form according to claim 1, **characterised in that** the active ingredient or active ingredients are pharmaceutical active ingredients which have been selected from the group consisting of opiates, opioids, tranquillisers, preferably benzodiazepines, stimulants and further narcotics.

3. A dosage form according to claim 2, **characterised in that** the active ingredients or active ingredients have been selected from the group consisting of N-{1-[2-(4-ethyl-5-oxo-2-tetrazolin-1-yl)ethyl]-4-methoxymethyl-4-piperidyl}propionanilide (alfentanil), 5,5-diallylbarbituric acid (allobarbital), allylprodine, alphaprodine, 8-chloro-1-methyl-6-phenyl-4*H-*[1,2,4]triazolo[4,3-a][1,4]-benzodiazepine (alprazolam), 2-diethylaminopropiophenone (amfepramone), (±)-α-methylphenethylamine (amphetamine), 2-(α-methylphenethylamino)-2-phenylacetonitrile (amphetaminil), 5-ethyl-5-isopentylbarbituric acid (amobarbital) anileridine, apocodeine, 5,5-diethylbarbituric acid (barbital), benzylmorphine, bezitramide, 7-bromo-5-(2-pyridyl)-1*H-*1,4-benzodiazepine-2(3*H*)-one (bromazepam), 2-bromo-4-(2-chlorophenyl)-9-methyl-6*H*-thieno[3,2-*f*]-[1,2,4]triazolo[4,3-a][1,4]diazepine (brotizolam), 17-cyclopropylmethyl-4,5α-epoxy-7α[(S)-1-hydroxy-1,2,2-trimethyl-propyl]-6-methoxy-6,14-*endo-*ethanomorphinan-3-ol (buprenorphine), 5-butyl-5-ethylbarbituric acid (butobarbital), butorphanol, (7-chloro-1,3-dihydro-1-methyl-2-oxo-5-phenyl-2*H*-1,4-benzodiazepin-3-yl) dimethylcarbamate (camazepam), (1*S*,2*S*)-2-amino-1-phenyl-1-propanol (cathine/D-norpseudoephedrine), 7-chloro-N-methyl-5-phenyl-3*H*-1,4-benzodiazepin-2-ylamine 4-oxide (chlordiazepoxide), 7-chloro-1-methyl-5-phenyl-1*H*-1,5-benzodiazepine-2,4(3*H*,5*H*)-dione (clobazam), 5-(2-chlorophenyl)-7-nitro-1*H*-1,4-benzodiazepin-2(3*H*)-one (clonazepam), clonitazene, 7-chloro-2,3-dihydro-2-oxo-5-phenyl-1*H*-1,4-benzodiazepine-3-carboxylic acid (clorazepate), 5-(2-chlorophenyl)-7-ethyl-1-methyl-1*H-*thieno[2,3-e][1,4]diazepin-2(3*H*)-one (clotiazepam), 10-chloro-11b-(2-chlorophenyl)-2,3,7,11b-tetrahydrooxazolo[3,2-d][1,4]benzodiazepin-6(5*H*)-one (cloxazolam), (-)-methyl-[3β-benzoyloxy-2β(1α*H*,5α*H*)-tropane carboxylate] (cocaine), 4,5α-epoxy-3-methoxy-17-methyl-7-morphinen-6α-ol (codeine), 5-(1-cyclohexenyl)-5-ethylbarbituric acid (cyclobarbital), cyclorphan, cyprenorphine, 7-chloro-5-(2-chlorophenyl)-1*H*-1,4-benzodiazepin-2(3*H*)-one (delorazepam), desomorphine, dextromoramide, (+)-(1-benzyl-3-dimethylamino-2-methyl-1-phenylpropyl) propionate (dextropropoxyphene), dezocine, diampromide, diamorphone, 7-chloro-1-methyl-5-phenyl-1*H*-1,4-benzodiazepin-2(3*H*)-one (diazepam), 4,5α-epoxy-3-methoxy-17-methyl-6α-morphinanol (dihydrocodeine), 4,5α-epoxy-17-methyl-3,6a-morphinandiol (dihydromorphine), dimenoxadol, dimephetamol, dimethylthiambutene, dioxaphetyl butyrate, dipipanone, (6a*R*, 10a*R*)-6,6,9-trimethyl-3-pentyl-6a,7,8,10a-tetrahydro-6*H*-benzo[c]chromen-1-ol (dronabinol), eptazocine, 8-chloro-6-phenyl-4*H*-[1,2,4]triazolo-[4,3-*a*][1,4]benzodiazepine (estazolam), ethoheptazine, ethylmethylthiambutene, ethyl [7-chloro-5-(2-fluorophenyl)-2,3-dihydro-2-oxo-1*H*-1,4-benzodiazepine-3-carboxylate] (ethyl loflazepate), 4,5α-epoxy-3-ethoxy-17-methyl-7-morphinen-6α-ol (ethylmorphine), etonitazene, 4,5α-epoxy-7α-(1-hydroxy-1-methylbutyl)-6-methoxy-17-methyl-6,14-endo-etheno-morphinan-3-ol (etorphine), *N*-ethyl-3-phenyl-8,9,10-trinorbornan-2-ylamine (fencamfamine), 7-[2-(α-methylphenethylamino)-ethyl]-theophylline) (fenethylline), 3-(α-methylphenethylamino)propionitrile (fenproporex), *N*-(1-phenethyl-4-piperidyl)propionanilide (fentanyl), 7-chloro-5-(2-fluorophenyl)-1-methyl-1*H*-1,4-benzodiazepin-2(3*H*)-one (fludiazepam), 5-(2-fluorophenyl)-1-methyl-7-nitro-1*H*-1,4-benzodiazepin-2(3*H*)-one (flunitrazepam), 7-chloro-1-(2-diethylaminoethyl)-5-(2-fluorophenyl)-1*H*-1,4-benzodiazepin-2(3*H*)-one (flurazepam), 7-chloro-5-phenyl-1-(2,2,2-trifluoroethyl)-1*H*-1,4-benzodiazepin-2(3*H*)-one (halazepam), 10-bromo-11b-(2-fluorophenyl)-2,3,7,11b-tetrahydro[1,3]oxazolo[3,2-d][1,4]benzodiazepin-6(5*H*)-one (haloxazolam), heroin, 4,5α-epoxy-3-methoxy-17-methyl-6-morphinanone (hydrocodone), 4,5α-epoxy-3-hydroxy-17-methyl-6-morphinanone (hydromorphone), hydroxypethidine, isomethadone, hydroxymethylmorphinan, 11-chloro-8,12b-dihydro-2,8-dimethyl-12b-phenyl-4*H*-[1,3]oxazino[3,2-*d*] [1,4] benzodiazepine-4,7(6*H*)-dione (ketazolam), 1-[4-(3-hydroxyphenyl)-1-methyl-4-piperidyl]-1-propanone (ketobemidone), (3*S*,6*S*)-6-dimethylamino-4,4-diphenylheptan-3-yl acetate (levacetylmethadol (LAAM)), (-)-6-dimethylamino-4,4-diphenol-3-heptanone (levomethadone), (-)-17-methyl-3-morphinanol (levorphanol), levophenacylmorphane, lofentanil, 6-(2-chlorophenyl)-2-(4-methyl-1-piperazinylmethylene)-8-nitro-2*H-*imidazo[1,2-*a*] [1,4]-benzodiazepin-1(4*H*)-one (loprazolam), 7-chloro-5-(2-chlorophenyl)-3-hydroxy-1*H*-1,4-benzodiazepin-2(3*H*)-one (lorazepam), 7-chloro-5-(2-chlorophenyl)-3-hydroxy-1-methyl-1*H*-1,4-benzodiazepin-2(3*H*)-one (lormetazepam), 5-(4-chlorophenyl)-2,5-dihydro-3*H*-imidazo[2,1-a]isoindol-5-ol (mazindol), 7-chloro-2,3-dihydro-1-methyl-5-phenyl-1*H-*1,4-benzodiazepine (medazepam), *N*-(3-chloropropyl)-α-methylphenethylamine (mefenorex), meperidine, 2-methyl-2-propyltrimethylene dicarbamate (meprobamate), meptazinol, metazocine, methylmorphine, N,α-dimethylphenethylamine (metamphetamine), (±)-6-dimethylamino-4,4-diphenol-3-heptanone (methadone), 2-methyl-3-o-tolyl-4(3*H*)-quinazolinone (methaqualone), methyl [2-phenyl-2-(2-piperidyl)acetate] (methylphenidate), 5-ethyl-1-methyl-5-phenylbarbituric acid (methylphenobarbital), 3,3-diethyl-5-methyl-2,4-piperidinedione (methyprylon), metopon, 8-chloro-6-(2-fluorophenyl)-1-methyl-4*H*-imidazo[1,5-a]-[1,4]benzodiazepine (midazolam), 2-(benzhydrylsulfinyl)acetamide (modafinil), 4,5α-epoxy-17-methyl-7-morphinen-3,6α-diol (morphine), myrophine, (±)-*trans*-3-(1,1-dimethylheptyl)-7,8,10,10α-tetrahydro-1-hydroxy-6,6-dimethyl-6*H*-dibenzo-[*b,d*]pyran-9(6α*H*)-one (nabilone), nalbuphene, nalorphine, narceine, nicomorphine, 1-methyl-7-nitro-5-phenyl-1*H*-1,4-benzodiazepin-2(3*H*)-one (nimetazepam), 7-nitro-5-phenyl-1*H*-1,4-benzodiazepin-2(3*H*)-one (nitrazepam), 7-chloro-5-phenyl-1*H*-1,4-benzodiazepin-2(3*H*)-one (nordazepam), norlevorphanol, 6-dimethylamino-4,4-diphenyl-3-hexanone (normethadone), normorphine, norpipanone, the exudation from plants belonging to the species *Papaver somniferum* (opium), 7-chloro-3-hydroxy-5-phenyl-1*H*-1,4-benzodiazepin-2(3*H*)-one (oxazepam), (*cis-trans*)-10-chloro-2,3,7,11b-tetrahydro-2-methyl-11b-phenyloxazolo[3,2-d]-[1,4]benzodiazepin-6-(5*H*)-one (oxazolam), 4,5α-epoxy-14-hydroxy-3-methoxy-17-methyl-6-morphinanone (oxycodone), oxymorphone, plants and parts of plants belonging to the species *Papaver somniferum* (including the subspecies *setigerum) (Papaver somniferum),* papaveretum, 2-imino-5-phenyl-4-oxazolidinone (pernoline), 1,2,3,4,5,6-hexahydro-6,11-dimethyl-3-(3-methyl-2-butenyl)-2,6-methano-3-benzazocin-8-ol (pentazocine), 5-ethyl-5-(1-methylbutyl)-barbituric acid (pentobarbital), ethyl (1-methyl-4-phenyl-4-piperidinecarboxylate) (pethidine), phenadoxone, phenomorphane, phenazocine, phenoperidine, piminodine, pholcodeine, 3-methyl-2-phenylmorpholine (phenmetrazine), 5-ethyl-5-phenylbarbituric acid (phenobarbital), α,α-dimethylphenethylamine (phentermine), 7-chloro-5-phenyl-1-(2-propynyl)-1*H-*1,4-benzodiazepin-2(3*H*)-one (pinazepam), α-(2-piperidyl)benzhydryl alcohol (pipradrol), 1'-(3-cyano-3,3-diphenylpropyl)[1,4'-bipiperidine]-4'-carboxamide (piritramide), 7-chloro-1-(cyclopropylmethyl)-5-phenyl-1*H*-1,4-benzodiazepin-2(3*H*)-one (prazepam), profadol, proheptazine, promedol, properidine, propoxyphene, N-(1-methyl-2-piperidinoethyl)-N-(2-pyridyl)propionamide, methyl {3-[4-methoxycarbonyl-4-(*N*-phenylpropanamido)piperidino]propanoate} (remifentanil), 5-sec-butyl-5-ethylbarbituric acid (secbutabarbital), 5-allyl-5-(1-methylbutyl)-barbituric acid (secobarbital), *N*-{4-methoxymethyl-1-[2-(2-thienyl)ethyl]-4-piperidyl}propionanilide (sufentanil), 7-chloro-2-hydroxy-methyl-5-phenyl-1*H-*1,4-benzodiazepin-2(3*H*)-one (temazepam), 7-chloro-5-(1-cyclohexenyl)-1-methyl-1*H*-1,4-benzodiazepin-2(3*H*)-one (tetrazepam), ethyl (2-dimethylamio-1-phenyl-3-cyclohexene-1-carboxylate) (tilidine, cis and *trans*))*,* tramadol, 8-chloro-6-(2-chlorophenyl)-1-methyl-4*H-*[1,2,4]triazolo[4,3-a][1,4]benzodiazepine (triazolam), 5-(1-methylbutyl)-5-vinylbarbituric acid (vinylbital), (1R*,2R*)-3-(3-dimethylamino-1-ethyl-2-methyl-propyl)-phenol, (1R,2R,4S)-2-(dimethylamino)methyl-4-(p-fluorobenzyloxy)-1-(m-methoxyphenyl)cyclohexanol, in each case optionally in the form of corresponding stereoisomeric compounds and corresponding derivatives, in particular esters or ethers, and in each case physiologically acceptable compounds, in particular salts and solvates.

4. A dosage form according to claim 2 or claim 3, **characterised in that** the stimulant has been selected from the group consisting of amphetamine, norpseudoephedrine, methylphenidate and in each case optionally the corresponding physiologically acceptable compounds thereof, in particular the bases, salts and solvates thereof.

5. A dosage form according to any one of claims 1 to 4, **characterised in that** the emetic is based on one or more constituents of ipecacuanha (ipecac) root, preferably on the constituent emetine, and/or is apomorphine.

6. A dosage form according to any one of claims 1 to 5, **characterised in that** both of the subunits (a) and (b) assume multiparticulate form, preferably the form of microtablets, microcapsules, micropellets, granules, spheroids, beads or pellets, optionally press-moulded to form tablets, packaged in capsules or suspended in a liquid or a gel, wherein the same form, i.e. shape, is selected for both of subunits (a) and (b).

7. A dosage form according to claim 6, **characterised in that** the particular largely identically shaped multiparticulate forms of subunits (a) and (b) are also visually indistinguishable from one another.

8. A dosage form according to any one of claims 1 to 5, **characterised in that** subunits (a) and (b) are in each case arranged in layers relative to one another.

9. A dosage form according to claim 8, **characterised in that** the layered subunits (a) and (b) are arranged vertically or horizontally relative to one another.

10. A dosage form according to claim 8, **characterised in that** subunit (b) forms a core, which is completely enclosed by subunit (a).

11. A dosage form according to claim 8, **characterised in that** subunit (a) forms a core, which is enclosed by subunit (b), wherein this enclosure comprises at least one channel which leads from the core to the surface of the dosage form.

12. A dosage form according to any one of claims 8 to 11, **characterised in that** at least one optionally swellable separation layer (c) is arranged between the layers of subunits (a) and (b).

13. A dosage form according to claim 8, 9 or 12, **characterised in that** it assumes the form of a tablet.

14. A dosage form according to any one of claims 8, 9, 12 or 13, **characterised in that** the entirety of the free surface of subunit (b) and optionally at least part of the free surface of subunit (a) and optionally at least part of the free surface of the optionally present separation layer (c) is coated with at least one barrier layer (d) which prevents release of the emetic.

15. A dosage form according to any one of claims 8, 9, 12 or 13, **characterised in that**, between subunits (a) and (b), it comprises a push layer (p) and all the free surfaces of the layer structure comprising subunits (a) and (b), the push layer (p) and optionally the separation layer (c) are provided with a semipermeable coating (e), which is permeable to the release medium, but is substantially impermeable to the active ingredient and the emetic, and wherein this coating (e) comprises at least one opening for release of the active ingredient in the area of subunit (a).

16. A dosage form according to any one of claims 1 to 5, **characterised in that** subunit (a) assumes the form of a tablet, the edge face and optionally one of the two main faces of which is covered with a barrier layer (d) containing the emetic.

17. A dosage form according to any one of claims 1 to 16, **characterised in that** it comprises at least one active ingredient at least partially in delayed-release form.

18. A dosage form according to any one of claims 1 to 17 for oral administration.

19. A dosage form according to claim 18, **characterised in that** it comprises at least one coating resistant to gastric juices.

## Revendications

1. Forme d'administration solide assurée contre un usage abusif, comprenant au moins une substance active avec un potentiel d'usage abusif et au moins un émétique séparé dans l'espace de celle-ci, la ou les substances actives se trouvant dans au moins une sous-unité (a) et l'émétique dans au moins une sous-unité (b) et l'émétique n'étant quasiment pas libéré de la sous-unité (b) lors d'une administration conforme aux dispositions en vigueur de la forme d'administration dans le corps.

2. Forme d'administration selon la revendication 1, **caractérisée en ce que** la ou les substances actives sont des substances actives pharmaceutiques qui sont choisies dans le groupe formé par les opiacés, les opioïdes, les tranquillisants, de préférence les benzodiazépines, les stimulants et d'autres anesthésiants.

3. Forme d'administration selon la revendication 2, **caractérisée en ce que** la ou les substances actives ont été choisies dans le groupe constitué par les composés N-{1-[2-(4-éthyl-5-oxo-2-tétrazolin-1-yl)éthyl]-4-méthoxyméthyl-4-pipéridyl}propionanilide (Alfentanil), acide 5,5-diallylbarbiturique (Allobarbital), allylprodine, alphaprodine, 8-chloro-1-méthyl-6-phényl-4H-[1,2,4]triazolo[4,3-a][1,4]-benzodiazépine (Alprazolam), 2-diéthylaminopropiophénone (Amfepramon), (±)-α-méthylphénéthylamine (Amfetamin), 2-(α-méthylphénéthylamino)-2-phénylacétonitrile (Amfetaminil), acide 5-éthyl-5-isopentylbarbiturique (Amobarbital), aniléridine, apocodéine, acide 5,5-diéthylbarbiturique (Barbital), benzylmorphine, bézitramide, 7-bromo-5-(2-pyridyl)-1H-1,4-benzodiazépin-2(3H)-one (Bromazepam), 2-bromo-4-(2-chlorophényl)-9-méthyl-6H-thiéno[3,2-f][1,2,4]triazolo[4,3-a][1,4]diazépine (Brotizolam), 17-cyclopropylméthyl-4,5α-époxy-7α[(S)-1-hydroxy-1,2,2-triméthyl-propyl]-6-méthoxy-6,14-endo-éthanomorphinan-3-ol (Buprenorphin), acide 5-butyl-5-éthylbarbiturique (Butobarbital), Butorphanol, (7-chloro-1,3-dihydro-1-méthyl-2-oxo-5-phényl-2H-1,4-benzodiazépin-3-yl)-diméthyl-carbamate (Camazepam), (1S,2S)-2-amino-1-phényl-1-propanol (Cathin/D-Norpseudoephedrin), 4-oxyde de 7-chloro-N-méthyl-5-phényl-3H-1,4-benzodiazépin-2-ylamine (Chlordiazepoxid), 7-chloro-1-méthyl-5-phényl-1H-1,5-benzodiazépin-2,4(3H,5H)-dione (Clobazam), 5-(2-chlorophényl)-7-nitro-1H-1,4-benzodiazépin-2(3H)-one (Clonazepam), Clonitazen, acide 7-chloro-2,3-dihydro-2-oxo-5-phényl-1H-1,4-benzodiazépine-3-carboxylique (Clorazepat), 5-(2-chlorophényl)-7-éthyl-1-méthyl-1H-thiéno[2,3-e][1,4]diazépin-2(3H)-one (Clotiazepam), 10-chloro-11b-(2-chlorophényl)-2,3,7,11b-tétrahydrooxazolo[3,2-d][1,4]benzodiazépin-6(5H)-one (Cloxazolam), (-)-[3β-benzoyloxy-2β(1αH,5αH)-tropanecarboxylate] de méthyle (Cocaïne), 4,5α-époxy-3-méthoxy-17-méthyl-7-morphinén-6α-ol (Codéine), acide 5-(1-cyclohexényl)-5-éthylbarbiturique (Cyclobarbital), Cyclorphane, Cyprenorphine, 7-chloro-5-(2-chlorophényl)-1H-1,4-benzodiazépin-2(3H)-one (Delorazepam), Desomorphine, Dextromoramide, propionate de (+)-(1-benzyl-3-diméthylamino-2-méthyl-1-phénylpropyle) (Dextropropoxyphen), Dezocine, Diampromide, Diamorphone, 7-chloro-1-méthyl-5-phényl-1H-1,4-benzodiazépin-2(3H)-one (Diazepam), 4,5α-époxy-3-méthoxy-17-méthyl-6α-morphinanol (Dihydrocodéine), 4,5 α -époxy-17-méthyl-3,6a-morphinanediol (Dihydromorphine), Dimenoxadol, Dimephetamol, Diméthylthiambutène, butyrate de dioxaphétyle, dipipanone, (6aR,10aR)-6,6,9-triméthyl-3-pentyl-6a,7,8,10a-tétrahydro-6H-benzo[c]chromén-1-ol (Dronabinol), Eptazocine, 8-chloro-6-phényl-4H-[1,2,4]triazolo[4,3-a] [1,4] benzodiazépine (Estazolam), éthoheptazine, éthylméthylthiambutène, [7-chloro-5-(2-fluorophényl)-2,3-dihydro-2-oxo-1H-1,4-benzodiazépine-3-carboxylate] d'éthyle (Ethylloflazepat), 4,5 α-époxy-3-éthoxy-17-méthyl-7-morphinén-6a -ol (Ethylmorphine), étonitazène, 4,5α-époxy-7α-(1-hydroxy-1-méthylbutyl)-6-méthoxy-17-méthyl-6,14-endo-éthénomorphinan-3-ol (Etorphin), N-éthyl-3-phényl-8,9,10-trinorbornan-2-ylamine (Fencamfamin), 7-[2-(α-méthylphénéthylamino)éthyl]-théophylline) (Fenetyllin), 3-(α-méthylphénéthylamino)propionitrile (Fenproporex), N-(1-phénéthyl-4-pipéridyl)propionanilide (Fentanyl), 7-chloro-5-(2-fluorophényl)-1-méthyl-1H-1,4-benzodiazépin-2(3H)-one (Fludiazepam), 5-(2-fluorophényl)-1-méthyl-7-nitro-1H-1,4-benzodiazépin-2(3H)-one (Flunitrazepam), 7-chloro-1- (2-diéthylaminoéthyl)-5-(2-fluorophényl)-1H-1,4-benzodiazépin-2(3H)-one (Flurazepam), 7-chloro-5-phényl-1-(2,2,2-trifluoroéthyl)-1H-1,4-benzodiazépin-2(3H)-one (Halazepam), 10-bromo-11b-(2-fluorophényl)-2,3,7,11b-tétrahydro[1,3]oxazolo[3,2-d][1,4]benzodiazépin-6(5H)-one (Haloxazolam), héroïne, 4,5α-époxy-3-méthoxy-17-méthyl-6-morphinanone (Hydrocodon), 4,5α-époxy-3-hydroxy-17-méthyl-6-morphinanone (Hydromorphon), hydroxypéthidine, isométhadone, hydroxyméthylmorphinane, 11-chloro-8,12b-dihydro-2,8-diméthyl-12b-phényl-4H-[1,3]oxazino[3,2-d][1,4]benzodiazépin-4,7(6H)-dione (Ketazolam), 1- [4-(3-hydroxyphényl)-1-méthyl-4-pipéridyl]-1-propanone (Ketobemidon), acétate de (3S,6S)-6-diméthylamino-4,4-diphénylheptan-3-yle (Levacetylmethadol (LAAM)), (-)-6-diméthylamino-4,4-diphényl-3-heptanone (Levométhadon), (-)-17-méthyl-3-morphinanol (Levorphanol), lévophénacylmorphane, Lofentanil, 6-(2-chlorophényl)-2-(4-méthyl-1-pipérazinylméthylène)-8-nitro-2H-imidazo[1,2-*a*][1,4]-benzodiazépin-1(4H)-one (Loprazolam), 7-chloro-5-(2-chlorophényl)-3-hydroxy-1H-1,4-benzodiazépin-2(3H)-one (Lorazepam), 7-chloro-5-(2-chlorophényl)-3-hydroxy-1-méthyl-1H-1,4-benzodiazépin-2(3H)-one (Lormetazepam), 5-(4-chlorophényl)-2,5-dihydro-3H-imidazo[2,1-a]isoindol-5-0l (Mazindol), 7-chloro-2,3-dihydro-1-méthyl-5-phényl-1H-1,4-benzodiazépine (Medazepam), N-(3-chloropropyl)-α-méthylphénéthylamine (Mefenorex), Meperidine, 2-méthyl-2-propyltriméthylènedicarbamate (Meprobamat), Meptazinol, Metazocin, méthylmorphine, N,α-diméthylphénéthylamine (Metamfetamin), (±)-6-diméthylamino-4,4-diphényl-3-heptanone (méthadone), 2-méthyl-3-o-toluyl-4(3H)-quinazolinone (méthaqualone), [2-phényl-2-(2-pipéridyl)acétate] de méthyle (Methylphenidat), acide 5-éthyl-1-méthyl-5-phénylbarbiturique (Methylphenobarbital), 3,3-diéthyl-5-méthyl-2,4-pipéridinedione (Methyprylon), Metopon, 8-chloro-6-(2-fluorophényl)-1-méthyl-4H-imidazo[1,5-a][1,4]benzodiazépine (Midazolam), 2-(benzhydrylsulfinyl)acétamide (Modafinil), 4,5α-époxy-17-méthyl-7-morphinène-3,6a-diol (Morphine), Myrophine, (±)-trans-3-(1,1-diméthylheptyl)-7,8,10,10α-tétrahydro-1-hydroxy-6,6-diméthyl-6H-dibenzo[b,d]pyrann-9(6αH)-one (Nabilon), Nalbuphen, Nalorphine, Narceine, Nicomorphine, 1-méthyl-7-nitro-5-phényl-1H-1,4-benzodiazépin-2(3H)-one (Nimetazepam), 7-nitro-5-phényl-1H-1,4-benzodiazépin-2(3H)-one (Nitrazepam), 7-chloro-5-phényl-1H-1,4-benzodiazépin-2(3H)-one (Nordazepam), Norlevorphanol, 6-diméthylamino-4,4-diphényl-3-hexanone (Norméthadon), Normorphine, Norpipanone, le jus qui s'écoule des végétaux appartenant au type Papaver somniferum (Opium), 7-chloro-3-hydroxy-5-phényl-1H-1,4-benzodiazépin-2(3H)-one (Oxazepam), (cis-trans)-10-chloro-2,3,7,11b-tétrahydro-2-méthyl-11b-phényloxazolo[3,2-d][1,4]benzodiazépin-6-(5H)-one (Oxazolam), 4,5α-époxy-14-hydroxy-3-méthoxy-17-méthyl-6-morphinanone (Oxycodon), Oxymorphone, les végétaux et les parties de végétaux des plantes appartenant au type Papaver somniferum (y compris la sous-espèce setigerum) (Papaver somniferum), Papaveretum, 2-imino-5-phényl-4-oxazolidinone (Pernolin), 1,2,3,4,5,6-hexahydro-6,11-diméthyl-3-(3-méthyl-2-butényl)-2,6-méthano-3-benzazocin-8-ol (pentazocine), acide 5-éthyl-5-(1-méthylbutyl)-barbiturique (pentobarbital), (1-méthyl-4-phényl-4-pipéridincarboxylate) d'éthyle (Pethidine), Phenadoxon, Phenomorphan, Phenazocine, Phenoperidine, Piminodine, Pholcodeine, 3-méthyl-2-phénylmorpholine (Phenmetrazine), acide 5-éthyl-5-phénylbarbiturique (phénobarbital), α,α-diméthylphénéthylamine (Phentermin), 7-chloro-5-phényl-1-(2-propynyl)-1H-1,4-benzodiazépin-2(3H)-one (Pinazepam), alcool α-(2-pipéridyl)benzhydrylique (Pipradrol), 1'-(3-cyano-3,3-diphénylpropyl)[1,4'-bipipéridine]-4'-carboxamide (Piritramid), 7-chloro-1-(cyclopropylméthyl)-5-phényl-1H-1,4-benzodiazépin-2(3H)-one (Prazepam), Profadol, Proheptazine, Promedol, Properidine, Propoxyphen, N-(1-méthyl-2-pipéridinoéthyl)-N-(2-pyridyl)propionamide, {3-[4-méthoxycarbonyl-4-(N-phénylpropanamido)pipéridino]propanoate} de méthyle (Remifentanil), acide 5-sec-butyl-5-éthylbarbiturique (Secbutabarbital), acide 5-allyl-5-(1-méthylbutyl)-barbiturique (Secobarbital), N-{4-méthoxyméthyl-1-[2-(2-thiényl)éthyl]-4-pipéridyl}propionanilide (Sufentanil), 7-chloro-2-hydroxy-méthyl-5-phényl-1H-1,4-benzodiazépin-2(3H)-one (Temazepam), 7-chloro-5-(1-cyclohexényl)-1-méthyl-1H-1,4-benzodiazépin-2(3H)-one (Tetrazepam), (2-diméthylamino-1-phényl-3-cyclohexène-1-carboxylate) d'éthyle (Tilidin (cis et trans)), Tramadol, 8-chloro-6-(2-chlorophényl)-1-méthyl-4H-[1,2,4]triazolo[4,3-a][1,4]benzodiazépine (Triazolam), acide 5-(1-méthylbutyl)-5-vinylbarbiturique (Vinylbital), (1R*,2R*)-3-(3-diméthylamino-1-éthyl-2-méthyl-propyl)-phénol, (1R,2R,4S)-2-[diméthylamino)méthyl-4-(p-fluorobenzyloxy)-1-(m-méthoxyphényl)cyclohexanol, à chaque fois, le cas échéant, sous forme des composés stéréo-isomères correspondants ainsi que les dérivés correspondants, en particulier les esters ou les éthers et à chaque fois des composés physiologiquement acceptables, en particulier les sels et les solvates.

4. Forme d'administration selon la revendication 2 ou 3, **caractérisée en ce que** la substance stimulante a été choisie dans le groupe formé par amphétamine, norpseudoéphédrine, phénidate de méthyle et à chaque fois le cas échéant leurs composés physiologiquement acceptables correspondants, en particulier leurs bases, sels et solvates.

5. Forme d'administration selon l'une quelconque des revendications 1 à 4, **caractérisée en ce que** l'émétique est basé sur un ou plusieurs constituants du Radix Ipecacuanhae (racine d'ipécacuanha), de préférence le constituant émétine et/ou est l'apomorphine.

6. Forme d'administration selon l'une quelconque des revendications 1 à 5, **caractérisée en ce que** les deux sous-unités (a) et (b) se trouvent, sous forme microparticulaire, de préférence sous forme de microcomprimés, de microcapsules, de micropellets, de granulats, de sphères, de billes ou de pellets, le cas échéant, sous forme pressée en comprimés, transvasée dans des capsules ou en suspension dans un liquide ou un gel, en choisissant tant pour la sous-unité (a) que pour la sous-unité (b) la même forme c'est-à-dire le même aspect.

7. Forme d'administration selon la revendication 6, **caractérisée en ce que** les formes multiparticulaires à chaque fois dans une large mesure identiques de la sous-unité (a) ou (b) ne peuvent pas non plus être distinguées visuellement.

8. Forme d'administration selon l'une quelconque des revendications 1 à 5, **caractérisée en ce que** les sous-unités (a) et (b) sont assemblées l'une avec l'autre en forme de couches.

9. Forme d'administration selon la revendication 8, **caractérisée en ce que** les sous-unités (a) et (b) en forme de couches sont assemblées horizontalement ou verticalement l'une par rapport à l'autre.

10. Forme d'administration selon la revendication 8, **caractérisée en ce que** la sous-unité (b) forme un noyau qui est complètement enrobé par la sous-unité (a).

11. Forme d'administration selon la revendication 8, **caractérisée en ce que** la sous-unité (a) forme un noyau qui est entouré par la sous-unité (b), cet enrobage présentant au moins un canal qui conduit du noyau à la surface de la forme d'administration.

12. Forme d'administration selon l'une quelconque des revendications 8 à 11, **caractérisée en ce qu'**entre les couches des sous-unités (a) et (b) est disposée au moins une couche de séparation (c) le cas échéant gonflable.

13. Forme d'administration selon la revendication 8, 9 ou 12, **caractérisée en ce qu'**elle se trouve sous forme d'un comprimé.

14. Forme d'administration selon l'une quelconque des revendications 8, 9, 12 ou 13, **caractérisée en ce que** la surface libre de la sous-unité (b) est revêtue complètement et, le cas échéant, au moins une partie de la surface libre de la sous-unité (a) et le cas échéant au moins une partie de la surface libre de la couche de séparation le cas échéant présente (c) sont revêtues par au moins un couche formant une barrière (d) empêchant la libération de l'émétique.

15. Forme d'administration selon l'une quelconque des revendications 8, 9, 12 ou 13, **caractérisée en ce qu'**elle présente, entre les sous-unités (a) et (b) une couche à effet poussant (p) et toutes les surfaces libres de la structure à couches des sous-unités (a) et (b), de la couche à effet poussant (p) et le cas échéant de la couche de séparation (c) un revêtement (e) semi-perméable, qui est perméable à l'agent de libération, mais essentiellement imperméable pour la substance active et l'émétique et ce revêtement (e) présentant dans la zone de la sous-unité (a) au moins une ouverture pour la libération de la substance active.

16. Forme d'administration selon l'une quelconque des revendications 1 à 5, **caractérisée en ce que** la sous-unité (a) a la forme d'un comprimé dont l'âme et le cas échéant une des deux surfaces de base sont revêtues par au moins une couche formant une barrière (d) contenant l'émétique.

17. Forme d'administration selon l'une quelconque des revendications 1 à 16, **caractérisée en ce qu'**elle présente au moins une substance active qui se trouve au moins partiellement sous une forme de retard.

18. Forme d'administration selon l'une quelconque des revendications 1 à 17 destinée à une administration par voie orale.

19. Forme d'administration selon la revendication 18, **caractérisée en ce qu'**elle présente au moins un revêtement résistant au suc gastrique.
